Europäisches Patentamt

European Patent Office

Office européen des brevets

(11) Veröffentlichungsnummer: **0 010 676**
**B1**

(12) **EUROPÄISCHE PATENTSCHRIFT**

(19)

(12)

(45) Veröffentlichungstag der Patentschrift:
14.10.81

(21) Anmeldenummer: 79103968.8

(22) Anmeldetag: 15.10.79

(51) Int. Cl.³: **C 07 C 21/18, C 07 C 17/34,**
**C 08 K 5/02**

(54) Verfahren zur Herstellung von 1,1-Difluor-2-chloräthylen sowie dessen Verwendung als Treibgas bzw. Treibmittel.

(30) Priorität: **27.10.78 DE 2846812**

(43) Veröffentlichungstag der Anmeldung:
**14.05.80 Patentblatt 80/10**

(45) Bekanntmachung des Hinweises auf die Patenterteilung:
**14.10.81 Patentblatt 81/41**

(84) Benannte Vertragsstaaten:
**DE FR GB IT NL**

(56) Entgegenhaltungen:
**FR-A-1 082 142**
**GB-A-979 670**
**US-A-2 709 181**

(73) Patentinhaber: **BAYER AG, Zentralbereich Patente,**
**Marken und Lizenzen, D-5090 Leverkusen 1, Bayerwerk**
**(DE)**

(72) Erfinder: **Mitschke, Karl-Heinz, Dr., Am Berg 22,**
**D-5068 Odenthal (DE)**
Erfinder: **Niederprüm, Hans, Dr., Hofstrasse 27,**
**D-4019 Monheim (DE)**

# Verfahren zur Herstellung von 1,1-Difluor- 2-chloräthylen sowie dessen Verwendung als Treibgas bzw. Treibmittel

Die vorliegende Erfindung betrifft ein verbessertes Verfahren zur Herstellung von 1,1-Difluor-2-chloräthylen.

1,1-Difluor-2-chloräthylen, eine an sich seit langem bekannte Substanz, ist vielseitig einsetzbar; so zum Beispiel als Zwischenprodukt für organische Synthesen, als monomerer Baustein für fluorhaltige Polymere oder als mögliches Treibmittel für den Aerosol-Sektor.

Die Herstellung von 1,1-Difluor-2-chloräthylen erfolgte bisher durch relativ aufwendige Verfahren. So ist es zum Beispiel bekannt, 1,1-Difluor-2-chloräthylen durch Umsetzung von 1,1-Difluor-1,2,2-trichloräthan mit Zink in organischen Lösungsmitteln herzustellen (vgl. z. B. DE-PS 907 173, GB-PS 756 027, Am. Soc. 58, 402/1936). Dieses Verfahren ist sowohl von der Herstellung des Ausgangsmaterials als auch von der Aufarbeitung der entstehenden Zinkhalogenide sehr aufwendig, so daß es für eine technische Durchführung praktisch nicht in Frage kommt. 1,1-Difluor-2-chloräthylen kann auch durch thermische Abspaltung von Halogenwasserstoff aus 1,1-Difluor-2,2-dichloräthan bzw. 1,1-Difluor-1,2-dichloräthan bei 550 bis 1100° C in Gegenwart von metallischem Nickel oder aber auch von Raschigringen erhalten werden (vgl. z. B. US-PS 2 628 989, GB-PS 774 125 oder Ind. Eng. Chem. 43, 1253/1951). Die bei diesem Verfahren auftretenden hohen Temperaturen sind im Zusammenhang mit dem bei der Reaktion freiwerdenden Chlorwasserstoff bei großtechnischen Anlagen problemhaft. Ferner haben solche Pyrolysereaktionen den Nachteil, daß eine Reihe unerwünschter Nebenprodukte entsteht. Aus FR-A 1 082 142, Beispiel 2 ist es bekannt, aus 1,1-Difluoro-1,2-dichloräthan und KOH in Gegenwart eines Emulgators 1,1-Difluoro-2-chloräthan herzustellen. Ohne Emulgator werden jedoch nur geringe Mengen an 1,1-Difluoro-2-chloräthan erhalten.

Aufgabe der vorliegenden Erfindung war es, ein einfaches und wirtschaftliches Verfahren zur Herstellung von 1,1-Difluor-2-chloräthylen bereitzustellen.

Gegenstand der vorliegenden Erfindung ist ein Verfahren zur Herstellung von 1,1-Difluor-2-chloräthylen durch Dehydrohalogenierung von 1,1-Difluor-1,2-dichloräthan, dadurch gekennzeichnet, daß 1,1-Difluor-1,2-dichloräthan mit basisch reagierenden Substanzen in Gegenwart von Wasser bei Temperaturen zwischen 50 und 130° C und unter erhöhtem Druck und in Abwesenheit von oberflächenaktiven Substanzen umgesetzt wird, wobei das entstehende 1,1-Difluor-2-chloräthylen unmittelbar aus dem Reaktionsgemisch entfernt wird.

Ein weiterer Gegenstand der vorliegenden Erfindung ist die Verwendung von 1,1-Difluor-2-chloräthylen als Treibgas bzw. Treibmittel.

Überraschenderweise hat es sich herausgestellt, daß entgegen der Lehre der US-PS 2 709 181 die Herstellung von 1,1-Difluor-2-chloräthylen auch ohne Zusatz von Emulgatoren in kurzer Zeit und mit hohen Ausbeuten gelingt, wenn die Umsetzung von 1,1-Difluor-1,2-dichloräthan und den basisch reagierenden Stoffen in Gegenwart von Wasser in einer Druckreaktion bei erhöhter Temperatur durchgeführt wird. Die erfindungsgemäße, in der flüssigen Phase stattfindende Reaktion zeigt den Vorteil, daß keinerlei Emulgatoren benötigt werden, daß ohne organische Lösungsmittel gearbeitet werden kann, und somit die Bildung unerwünschter Nebenprodukte vermieden wird, daß die Aufarbeitung relativ problemlos ist und daß eine sehr gute Raum-Zeit-Ausbeute erzielt wird.

Das erfindungsgemäße Verfahren wird in einer allgemeinen Ausführung so durchgeführt, daß in einem Druckbehälter, der mit den üblichen Zusätzen, wie z. B. Rührer, Druckkühler, Druckkonstanthalteventile usw. versehen ist, eine der Reaktionskomponenten vorgelegt und die andere, vorzugsweise der basisch wirkende Stoff, in Wasser gelöst oder suspendiert, zudosiert wird, nachdem der Autoklav zuvor vorzugsweise mit einem Inertgas wie beispielsweise Stickstoff unter einen Vordruck gesetzt wird. Das während der Reaktion entstehende 1,1-Difluor-2-chloräthylen wird kontinuierlich über das Druckkonstanthalteventil in auf ca. −70° C gekühlte Fallen kondensiert und nach dem Trocknen (z. B. mit Kalziumchlorid) destilliert. Die Destillation kann dabei unter Druck oder bei Normaldruck erfolgen. Eventuell im Sumpf verbleibendes Ausgangsmaterial kann gegebenenfalls erneut zur Reaktion gebracht werden.

Die erfindungsgemäße Umsetzung erfolgt bei Temperaturen von 50 bis 130° C und bei Drucken von 1 bis 15 bar Überdruck, vorzugsweise von 3 bis 8 bar. Das erfindungsgemäße Verfahren kann sowohl kontinuierlich — auch mehrstufig — als auch diskontinuierlich durchgeführt werden.

Als Dehydrohalogenierungsmittel eignen sich basisch reagierende anorganische und/oder organische Substanzen, wie z. B. Alkalioxide oder Hydroxide, wie Natrium- oder Kaliumoxide oder Hydroxide, Erdalkalioxide wie z. B. CaO oder Erdalkalihydroxide, Alkalicarbonate oder Alkaliphosphate oder Alkalicarboxylate, Ammoniak und Amine, wie z. B. Trialkyl-, Dialkyl- oder Monoalkylamine. Bevorzugt wird Natriumhydroxid eingesetzt. Es ist ohne weiteres möglich, auch Gemische von basisch reagierenden Substanzen einzusetzen, wie z. B. Natriumhydroxid und Amine, wobei es besonders vorteilhaft ist, die Amine nur in katalytischen Mengen einzusetzen.

Die Mengen an basisch reagierenden Substanzen betragen etwa 1,0 bis 3,0 Mol, vorzugsweise etwa 1,0 bis 1,5 Mol pro Mol eingesetztes 1,1-Difluor-1,2-dichloräthan.

Das als Ausgangsmaterial eingesetzte 1,1-Difluor-1,2-dichloräthan kann in technischer Qualität — wie es aus der Herstellung durch Fluorierung von Trichloräthylen erhalten wird — eingesetzt werden. Besondere Reinigungsschritte sind nicht erforderlich.

Wasser muß ein einer solchen Menge zugeführt werden, daß der basisch wirkende Stoff gelöst bzw. zumindest suspendiert werden kann.

Das erfindungsgemäße Verfahren soll nun anhand der folgenden Beispiele noch näher erläutert werden. Prozentangaben beziehen sich soweit nicht anders angegeben auf Gewichtsprozente. Die angegebenen Druckwerte beziehen sich auf Überdruck.

### Beispiel 1

Ein Autoklav, versehen mit Rührer, beheizbarem Druckkühler und Druckkonstanthalte-Ventil, wurde mit 269 g (2 Mol) 1,1-Difluor-1,2-dichloräthan und 100 g Wasser gefüllt, mit Stickstoff unter einen Druck von ca. 5 bar gesetzt und danach auf eine Temperatur von ca. 90° C gebracht. Danach wurde eine wäßrige Lösung von 96 g (2,4 Mol) Natriumhydroxid in 600 g Wasser zugepumpt. Das während ca. 1 Std. sich bildende Olefin wurde über das auf ca. 6 bar (nach ca. 0,5 Std. 4 bar) eingestellte Druckkonstanthalte-Ventil entgast, durch ein sich anschließendes mit Calciumchlorid gefülltes Trokkenrohr geleitet und in auf ca. −60° C gekühlte Fallen kondensiert. Am Ende der Reaktion wurde der Autoklav völlig entspannt. Es wurden 176 g Rohprodukt erhalten, das aus 173 g (88% d. Theorie) 1,1-Difluor-2-chloräthylen (Kp −18° C) und 3 g Ausgangsmaterial bestand. Die Ausbeute ist an sich noch höher, da bei ihrer Berechnung Umfüllverluste etc. nicht berücksichtigt wurden. Die Verbindungen wurden $^1$H- und $^{19}$F-NMR- sowie IR-spektroskopisch identifiziert.

### Beispiel 2

In einen Autoklaven wurden 134,5 g (1 Mol) 1,1-Difluor-1,2-dichloräthan, 159 g (1,5 Mol) Natriumcarbonat und 500 ml Wasser gefüllt und die Mischung unter Rühren auf ca. 100° C erhitzt. Nach ca. 1,5 Std. wurde der Autoklav in eine auf ca. −60° C gekühlte Falle entgast. Es wurden 83 g (84% d. Theorie, vgl. Bsp. 1), an 1,1-Difluor-2-chloräthylen erhalten.

### Beispiel 3

Entsprechend Beispiel 2 wurden aus 134,5 g (1 Mol) 1,1-Difluor-1,2-dichloräthan, 500 g Wasser und 61,7 g (1,1 Mol) Calciumoxid bei ca. 110° C 78 g (79% d. Theorie) an 1,1-Difluor-2-chloräthylen erhalten.

### Beispiel 4

Entsprechend Beispiel 1 wurde zu einer Mischung aus 403,5 g (3 Mol) 1,1-Difluor-1,2-dichloräthan, 31,8 g (0,25 Mol) Dimethylcyclohexylamin und 100 g Wasser, die auf ca. 80° C und einen Druck von 6 bar gebracht wurden, eine Lösung von 124 g (3,1 Mol) Natriumhydroxid in 500 g Wasser dosiert. Nach dem Abgasen über eine gekühlte Falle nach ca. 1 Std. wurden 269 g (91% der Theorie) an 1,1-Difluor-2-chloräthylen erhalten.

### Beispiel 5
(Vergleichsbeispiel bei Normaldruck)

In einen Dreihalskolben, versehen mit Rückflußkühler, Innenthermometer und Rührer wurden zu 134,5 g (1 Mol) siedendem 1,1-Difluor-1,2-dichloräthan eine Lösung von 48 g (1,2 Mol) Natriumhydroxid in 150 g Wasser getropft. Auch nach 10,5 Std. konnten nur Spuren von 1,1-Difluor-2-chloräthylen in einer an den Rückflußkühler angeschlossenen, auf ca. −70° C gekühlten Falle erhalten werden. Auch die Zugabe von Aminen brachte keinen Erfolg.

### Patentansprüche

1. Verfahren zur Herstellung von 1,1-Difluor-2-chloräthylen durch Dehydrohalogenierung von 1,1-Difluor-1,2-dichloräthan, dadurch gekennzeichnet, daß 1,1-Difluor-1,2-dichloräthan mit basisch reagierenden Substanzen in Gegenwart von Wasser bei Temperaturen zwischen 50 und 130° C und unter erhöhtem Druck und in Abwesenheit von oberflächenaktiven Substanzen umgesetzt wird, wobei das entstehende 1,1-Difluor-2-chloräthylen unmittelbar aus dem Reaktionsgemisch entfernt wird.

2. Verfahren nach Anspruch 1, dadurch gekennzeichnet, daß die Dehydrohalogenierung mit Natriumhydroxid, gegebenenfalls im Gemisch mit katalytischen Mengen an Aminen, als basisch reagierende Substanz, durchgeführt wird.

3. Verfahren nach Anspruch 1 oder 2, dadurch gekennzeichnet, daß die Dehydrohalogenierung bei einem Überdruck von 1 bis 15 bar durchgeführt wird.

4. Verfahren nach einem oder mehreren der Ansprüche 1 bis 3, dadurch gekennzeichnet, daß die basisch reagierenden Substanzen in Mengen von etwa 1 bis 3,0 Mol pro Mol 1,1-Difluor-1,2-dichloräthan eingesetzt werden.

5. Verwendung des nach einem oder mehreren der Ansprüche 1 bis 4 hergestellten 1,1-Difluor-2-chloräthylen als Treibgas bzw. Treibmittel.

### Claims

1. Process for the produktion of 1,1-difluoro-2-chloroethylene by dehydrohalogenating 1,1-difluoro-1,2-dichloroethane, characterised in that 1,1-difluoro-1,2-dichloroethane is reacted with basically reacting substanzes in the presence of water at temperatures between 50 and 130°C and under elevated pressure and in the absence of surface active substances, the 1,1-difluoro-2-chloroethylene which forms being directly removed from the reaction mixture.

2. Process according to Claim 1, characterised in that the dehydrohalogenation is conducted with sodium hydroxide, optionally in admixture with catalytic amounts of amines, as the basically reacting substance.

3. Process according to Claim 1 or 2, characterised in that the dehydrohalogenation is conducted under a superatmospheric pressure of 1 to 15 bars.

4. Process according to one or more of Claims 1 to 3, characterised in that the basically reacting substances are employed in amounts of approx. 1 to 3.0 moles per mole of 1,1-difluoro-1,2-dichloroethane.

5. Use of the 1,1-difluoro-2-chloroethylene produced according to one or more of Claims 1 to 4 as a propellant gas or propellant.

### Revendications

1. Procédé de produktion de 1,1-difluoro-2-chloréthylène par déshydrohalogénation de 1,1-difluoro-1,2-dichloréthane, caractérisé en ce qu'on fait réagir du 1,1-difluoro-1,2-dichloréthane avec des substances à réaction basique en présence d'eau à des températures comprises entre 50 et 130°C et sous pression élevée et en l'absence de substances tensio-actives, le 1,1-difluoro-2-chloréthylène produit étant immédiatement séparé du mélange réactionnel.

2. Procédé suivant la revendication 1, caractérisé en ce que la déshydrohalogénation est effectuée avec de l'hydroxyde de sodium, éventuellement en mélange avec des quantités catalytiques d'amines, comme substance à réaction basique.

3. Procédé suivant la revendication 1 ou 2, caractérisé en ce que la déshydrohalogénation est conduite à une pression monométrique de 1 à 15 bars.

4. Procédé suivant une ou plusieurs des revendications 1 à 3, caractérisé en ce que les substances à réaction basique sont utilisées en quantités d'environ 1 à 3,0 moles par mole de 1,1-difluoro-1,2-dichloréthane.

5. Utilisation du 1,1-difluoro-2-chloréthylène produit selon une ou plusieurs des revendications 1 à 4 comme gaz prolulseur ou comme porogène.